# EUROPEAN PATENT APPLICATION

(11) **EP 0 714 658 A1**
(43) Date of publication of application: **05.06.1996**
(21) Application number: 95914560.8
(22) Date of filing: 11.04.1995
(51) Int. Cl.: A61K 31/19, A61K 31/235

(54) **APOPTOSIS INDUCER**

(30) Priority: 14.04.1994 JP 75836/94
(71) Applicant: OTSUKA PHARMACEUTICAL CO., LTD., Chiyoda-ku Tokyo 101 (JP)
(72) Inventor: ICHIKAWA, Hiroyuki, Tokushima-shi Tokushima 770 (JP); ONO, Yukihisa, Itano-gun Tokushima 771-02 (JP); MASUI, Yoshihiro, Itano-gun Tokushima 771-02 (JP); ADACHI, Masakazu, Takasaki-shi Gunma 370 (JP)
(74) Representative: Hansen, Bernd, Dr. Dipl.-Chem.
(86) International application number: JP9500709
(87) International publication number: WO9528154

(57) **Abstract**

An apoptosis inducer containing at least either a phenanthrene derivative represented by general formula (1) or a salt thereof in a pharmacologically available dose as the active ingredient together with an innocuous pharmacological carrier. In said formula, R represents hydrogen or lower alkyl. The inducer can induce or promote apoptosis and is hence useful in the medicinal field as, for example, carcinostatic, remedy for autoimmune diseases, remedy for liver diseases such as hepatitis and hepatocirrhosis, and cancerous metastasis inhibitor.

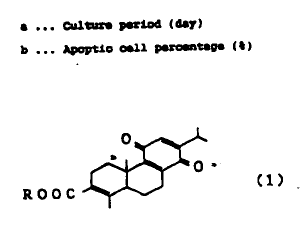

## Description

### Technical Field

The present invention relates to a novel apoptosis inducing composition.

### Disclosure of the Invention

The apoptosis inducing composition of this invention comprises, as an active ingredient, at least one member selected from the group consisting of phenanthrene derivatives represented by the following formula (1) 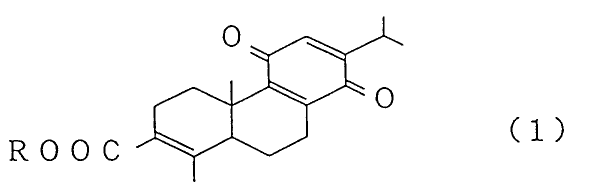
wherein R is a hydrogen atom or a lower alkyl group [hereinafter referred to as compound (1)], and salts thereof.

The above compound (1), and the process for production thereof are disclosed, for example, in Japanese Unexamined Patent Publications No. 211035/1992 and No. 1746/1994. It is also known that the compound is useful as an interleukin-1 inhibitor.

The inventor of this invention did further research into the compound (1) and found that the compound has apoptosis inducing (inducing or promoting) activity which is difficult to predict from said known action of the compound.

Meanwhile, it is said that two types of mechanism are involved in cell death. One is a classical type of cell death called necrosis. Morphologically, necrosis is characterized by marked swelling of mitochondria, swelling of cytoplasm and nuclear alteration, followed by cell destruction and autolysis. It occurs passively or incidentally. Tissue necrosis is generally caused by physical trauma to cells or a chemical poison, for instance.

Another type of cell death is called apoptosis (programmed cell death) [Kerr, J. F. R. and Wyllie, A. H., Br. J. Cancer, 265, 239 (1972)]. It is said that apoptosis can occur under various physiological conditions. Morphologically, apoptosis is characterized by loss of contact with neighboring cells, concentration of cytoplasm, endonuclease activity-associated chromatin condensation and pyknosis, and segmentation of the nucleus. Disappearance of microvilli from the cell surface and smoothening of the cell surface (vesicle formation on the cell surface: membrane blebbing) are also observed. Fragmentation of the nucleosome unit of DNA into DNA fragments 180-200 bases in size due to endonuclease activation is further observable. Final fragments of apoptotic body cells are phagocytosed by neighboring cells. This is the mechanism discussed by Duvall and Wyllie [Duvall, E. and Wyllie, A. H., Immunology Today, 7 (4), 115-119 (1986); Science, 245, 301-305 (1989)]. Wyllie further reported that glucocorticoid-induced apoptosis of thymocytes involves intracellular endonuclease activation [Wyllie, A. H., Nature, 284, 555-556 (1986)]. Endonuclease activity causes fragmentation, to the oligonucleotide level, of DNA in cells undergoing apoptosis and this can be readily confirmed by agarose gel electrophoresis.

Said apoptosis can be considered as preprogrammed cell death seen in the process of development, differentiation, or turnover of tissues [Wyllie, A. H., et al., Int. Rev. Cytol., 68, 251-306 (1980)].

In thymocytes, an increase in calcium concentration with the calcium ionophore or an increase in cAMP concentration leads to promotion of the DNA fragmentation which is characteristic of the above-mentioned apoptosis [Wyllie, A. H. et al., J. Pathol., 142, 67-77 (1984)] and, therefore, it is supposed that the calcium ion and/or cAMP be involved in the mechanisms of apoptosis. As an example so far reported, there may be mentioned apoptosis of HL-60 cells whose differentiation is induced by retinoic acid or calcium ionophore [Martin, S. J., et al., J. Immunol., 145, 1859-1867 (1990); Martin, S. J., et al., Clin. Exp. Immunol., 79, 448-453 (1990)].

Reportedly, said apoptosis not only occurs as physiological cell death in the process of embryogenesis and physiological death of normal cells in active cell cycle (e.g. liver, adrenal cortex and prostate cells), but also is induced by glucocorticoid treatment, cell injury by cytotoxic T cells, atrophy of hormone-dependent tissues, irradiation, NK cells, killer cells, tumor necrosis factor (TNF), lymphotoxin (LT), other cytokines, etc. [Wyllie, A. H. et al., Int. Rev. Cytol., 68, 251 (1980); Duvall, E. and Wyllie, A. H., Immunology Today, 7, 115-119 (1986); Sellins, K. S., et al., J. Immunol., 139, 3199 (1987); Yamada, T., et al., Int. J. Radiat. Biol., 53, 65 (1988); Wyllie, A. H., Nature, 284, 555 (1980); Schmid, D. S., et al., Proc. Natl. Acad. Sci. USA, 83, 1881-1885 (1986); John, C., et al., J. Immunol., 129 (4), 1782-1787 (1982); Howell, D. M., et al., J. Immunol., 140, 689-692 (1988); Gillian, B., et al., Eur. J. Immunol., 17, 689-693 (1987)].

In addition, apoptosis is also inducible by some antibodies, for example anti-CD3, anti-APO-I and anti-Fas antibodies [Trauth, B. C., et al., Science, 245, 301-305 (1989); Smith, C. A., et al. Nature, 337, 181-184 (1989); Tadakuma, T., et al., Eur. J. Immunol., 20, 779 (1990)] and, further, the induced apoptosis has been confirmed in the findings of Nakamura et al. as obtained in spontaneous regression of malignant tumor [Nakamura, Y., et al., Rinsho Hifuka (Jpn, J. Clin, Dermatol.), 35 (4), 289-295 (1981)].

There is a report describing apoptosis induction in acute leukemia cells by cycloheximide (a protein synthesis inhibitor), in small intestine crypt cells by actinomycin D (an RNA synthesis inhibitor), and in HL-60 cells by both [Martin, S. J., et al., J. Immunol., 145, 1859-1867 (1990)].

Recently, cancer treatment with anti-Apo-I antibody has been attempted as an apoptosis-related therapy. Among the myelodysplastic syndrome (MDS), in RAEB and RAEB in transformation (RAEB-t) in which blast cell growth is active, etoposide and aclarubicin are said to act as apoptosis promoting agents, which suppress blast cell growth [Shibuya, T., J. Clinical and Experimental Medicine, 160 (5), 319-323 (1992)].

Murakami et al. reported that about half of transgenic mice expressing anti-erythrocyte autoantibody manifest the autoimmune diseases as a result of loss of self tolerance and that this is due to deficiency in ability to eliminate the autoantibodies producing cells resulting from apoptosis induction by self antigen-autoantibody producing cells reactions as in normal mice [Murakami, M., et al., Nature, 357, 77-80 (1992)].

Watanabe-Fukunaga et al. suggest that, for MRL lpr/lpr mice, Fas moleculs relating to apoptosis has abnormality and the negative selection (apoptosis) mechanism of autoreactive T-cells does not work properly in thymus. Consequently, autoimmune disease occurs [Watanabe-Fukunaga, R., et al., Nature, 356, 314-317 (1992)].

On the other hand, it is known that, in the liver, mitogens induce the growth of hepatocytes to produce a hyperplastic state, and this state is normalized by falling off and death, i.e. apoptosis, of hepatocytes [Kerr, J. F., et al., Br. J. Cancer, 26, 239-257 (1972)].

It is considered that in the process of transition from chronic hepatitis to hepatic cirrhosis and further to hepatic cancer, apoptosis is in a controlled state and thus cytotoxic T cells can induce hepatocyte inflammation, followed by fibrosis, causing aggravation to hepatic cirrhosis and that, therefore, hepatitis might be suppressed and development into cirrhosis prevented when apoptosis is promoted.

According to the present invention, there is provided an apoptosis inducing composition comprising, as an active ingredient, a pharmacologically effective amount of at least one member selected from the group consisting of the compounds of the formula (1) and salts thereof, and a nontoxic pharmaceutically acceptable carrier therefor.

The apoptosis inducing composition of this invention has an ability to induce or promote apoptosis and, owing to this ability, is effective in the medicinal field, for example, as an anticancer agent, therapeutic agents for autoimmune diseases and for liver diseases such as hepatitis and hepatocirrhosis, a tumor metastasis inhibiting agent, etc. as mentioned hereinbefore.

The lower alkyl group in the formula (1) representing the compound (1) includes straight-chain or branched C₁₋₆ alkyl groups such as methyl, ethyl, propyl, isopropyl, butyl, tert-butyl, pentyl, hexyl and so on.

Among the species of compound (1) serving as the active ingredient, those having an acidic group can be easily converted to pharmaceutically acceptable salts with a basic compound. These salts can also be used as active ingredient compounds in the same manner as the free compounds. Examples of the foregoing basic compound are hydroxides, carbonates or hydrides of alkali metals or alkaline earth metals such as sodium hydroxide, potassium hydroxide, calcium hydroxide, sodium carbonate, potassium carbonate, sodium hydrogencarbonate, sodium hydride, etc. Also usable as the salt-forming basic compound are organic amines such as methylamine, ethylamine, isopropylamine, morpholine, piperazine, piperidine, 3,4-dimethoxyphenethylamine, etc. The reaction for forming a salt with the basic compound can be conducted in the conventional manner, for example, by the methods described in said publications.

The active ingredient compounds of the present invention include, of course, stereoisomers and optical isomers.

The compound (1) and salts thereof as the active ingredient for the apoptosis inducing composition according to the present invention are used in the form of the per se conventional pharmaceutical preparations. Such preparations are prepared using the conventional fillers, extenders, binding agents, moistening agents, disintegrating agents, surfactants, lubricants, and like diluents or excipients as nontoxic carriers. These pharmaceutical preparations may have various dosage forms selected according to the purposes of therapy, and typical examples thereof are tablets, pills, powders, solutions, suspensions, emulsions, granules, capsules, suppositories, injections (solutions, suspensions, etc.), eye-drops, etc.

For the manufacture of tablets, a wide variety of carriers so far well known in this field can be used. Thus, use can be made of, for example, vehicles or excipients such as lactose, sucrose, sodium chloride, glucose, urea, starch, calcium carbonate, kaolin, crystalline cellulose and silicic acid, binding agents such as water, ethanol, propanol, simple syrup, glucose solution, starch solution, gelatin solution, carboxymethylcellulose, shellac, methylcellulose, potassium phosphate and polyvinylpyrrolidone, disintegrating agents such as dry starch, sodium alginate, powdered agar, powdered laminaran, sodium hydrogencarbonate, calcium carbonate, polyoxyethylene sorbitan fatty acid esters, sodium lauryl sulfate, stearic acid monoglyceride, starch and lactose, disintegration inhibitors such as sucrose, stearin, cacao butter and hydrogenated oils, absorption promoters such as quaternary ammonium bases and sodium lauryl sulfate, wetting agents or humectants such as glycerol and starch, adsorbents such as starch, lactose, kaolin, bentonite and colloidal silica, and lubricants such as refined talc, stearic acid salts, powdered boric acid and polyethylene glycol. When necessary, the tablets may further be provided with a conventional coating to give, for example, sugar-coated tablets, gelatin-coated tablets, enteric-coated tablets, film-coated tablets, or double-coated or multilayer tablets.

For the manufacture of pills, a wide variety of carriers well known in the art can be used. Examples are excipients such as glucose, lactose, starch, cacao butter, hardened vegetable oils, kaolin and talc, binding agents such as powdered gum arabic, powdered tragacanth gum, gelatin and ethanol, and disintegrating agents such as laminaran and agar.

For the manufacture of suppositories, a wide variety of carriers so far known can be used. As examples, there may be mentioned polyethylene glycol, cacao butter, higher alcohols, higher alcohol esters, gelatin and semisynthetic glycerides.

In preparing injections, the solutions or suspensions are preferably sterilized and are preferably isotonic with blood and, for preparing such dosage forms as solutions, emulsions or suspensions, all the diluents in conventional use in this field can be employed. Thus, for example, water, ethyl alcohol, propylene glycol, ethoxylated isostearyl alcohol, polyoxylated isostearyl alcohol and polyoxyethylene sorbitan fatty acid esters may be mentioned. In this case, the pharmaceutical preparations may contain sodium chloride, glucose or glycerol in an amount sufficient to give isotonic solutions. It is possible to add conventional solubilizing agents, buffers, soothing agents, etc.

Furthermore, when necessary, the apoptosis inducing composition of the invention may contain coloring matters, preservatives, flavor enhancers, flavoring agents, sweetening agents and the like as well as other drugs.

The proportion of the active ingredient compound in the foregoing pharmaceutical composition of this invention is not critical but may suitably be selected from a wide range. Generally, however, the proportion is recommendably selected within the range of 1 to 70% by weight, preferably 1 to 30% by weight, based on the composition.

The route of administration of the above pharmaceutical preparations is not critical, but is selected according to the dosage form, the patient's age, sex and other factors and the severity of the disease to be treated. Thus, for instance, when they are provided in the form of tablets, pills, solutions, suspensions, emulsions, granules or capsules, the preparations are administered orally. Injectable solutions are administered intravenously, either alone or in admixture with conventional fluids for parenteral infusion containing glucose, amino acids and so on. Where necessary, these solutions may also be administered as it is by the intramuscular, intradermal, subcutaneous or intraperitoneal route. Suppositories are administered rectally. Of course, eye-drops are instilled into the eye.

While the dosage of the above pharmaceutical preparations is properly selected depending on the method of administration, the patient's age, sex and other background factors, severity of the disease and so on, it is generally recommended to administer about 0.1 to about 1000 mg, as the active ingredient, viz. compound (1), per kilogram body weight per day. The amount of the active ingredient to be contained in each dosage unit is about 1 to 600 mg.

The apoptosis inducing composition of the present invention can be applied to various diseases based on the apoptosis inducing or promoting activities thereof, and is expected to be pharmacologically effective as desired in the treatment of the diseases. These diseases include, for example, cancer, AIDS, ARC (AIDS related complex), ATL (adult T cell leukemia), hairy cell leukemia, myelopathy (HAM/TSP), respiratory disorder (HAB/HABA), arthropathy (HAAP), uveitis (HAU), other HTLV-I related diseases, autoimmune diseases such as SLE (systemic lupus erythematosus), collagen diseases such as rheumatoid arthritis (RA), ulcerative colitis, Sjögren's syndrome, primary biliary hepatic cirrhosis, idiopathic thrombocytopenic purpura (ITP), autoimmune hemolytic anemia, maysthenia gravis, Hashimoto's disease, and insulin dependent (type I) diabetes mellitus. The apoptosis inducing composition of the invention is also applicable to various diseases accompanied by thrombocytopenia, for example myelodysplastic syndrome, periodic thrombocytopenia, aplastic anemia, idiopathic thrombocytopenia, disseminated intravascular coagulation, etc. The composition of the invention is further applicable to various other diseases including various types of hepatitis (such as types C, A, B, and F), Alzheimer's disease, senile dementia of Alzheimer type, myocarditis, ARDS (adult respiratory distress syndrome), infectious diseases, liver cirrhosis, prostatic hypertrophy, uterine myoma, bronchial asthma, arteriosclerosis, various congenital malformations, nephritis, senile cataract, chronic fatigue syndrome, and myodystrophia.

The apoptosis inducing composition of the present invention, when administered as an anticancer composition, for instance, induces or promotes apoptosis of cancer cells and thereby produces an anticancer effect. In this case, the composition of the invention, irrespective of dosage form and/or route of administration, can be used in combination with one or more of various anticancer agents known as cancer chemotherapeutic agents and/or radiation therapy. The active ingredient compound of the invention which can produce an excellent anticancer effect can thus markedly promote the effect of the other anticancer agent or agents combinedly used to produce a synergistic effect. Therefore, even when the partner anticancer agent or agents are used in doses much smaller than the usual doses, a satisfactory anticancer effect can be obtained, whereby the adverse effects of the partner anticancer agent or agents can be minimized. As such chemotherapeutic agents, there may be mentioned, for example, 5-fluorouracil (5-FU; Kyowa Hakko Kogyo), mitomycin C (Kyowa Hakko Kogyo), futraful (FT-207; Taiho Pharmaceutical), endoxan (Shionogi & Co.) and toyomycin (Takeda Chemical Industries).

When used in the treatment of thrombocytopenia, the apoptosis inducing composition of the invention can inhibit blast cell multiplication in patients, for example, with MDS such as RAEB or RAEB-t, whereby proliferation of mature cells can be caused.

The apoptosis inducing composition of the present invention can induce or promote apoptosis, thereby treating hepatitis in patients with drug-induced hepatitis or viral hepatitis and preventing hepatocytes from fibrogenesis.

### Brief Description of the Drawings

Fig. 1 is a graph showing the results achieved by the apoptosis inducing efficacy test described in Pharmacological Test Example 1.

Fig. 2 is a graph showing the results achieved by the apoptosis inducing efficacy test described in Pharmacological Test Example 1.

Fig. 3 is a graph showing the results achieved by the apoptosis inducing efficacy test described in Pharmacological Test Example 1.

### Best Mode for Carrying Out the Invention

Given below are Preparation Examples illustrative of the preparation of the apoptosis inducing compositions according to the invention, and pharmacological tests on the active ingredient in the apoptosis inducing composition of the invention with the results set forth below.

A compound represented by the formula (A) 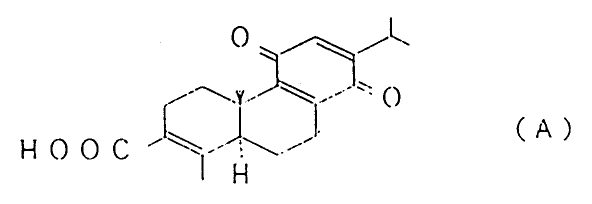
was used as one of the active ingredient compounds (hereinafter called "compound A").

### Preparation Example 1

| | |
|---|---|
| Compound A | 150 g |
| Avicel (trademark, Asahi Chemical Industry Co., Ltd.) | 40 g |
| Corn starch | 30 g |
| Magnesium stearate | 2 g |
| Hydroxypropylmethylcellulose | 10 g |
| Polyethylene glycol 6000 | 3 g |
| Castor oil | 40 g |
| Methanol | 40 g |

The above active ingredient compound, Avicel, corn starch and magnesium stearate were mixed and ground together and the resulting mixture was compression-molded with a dragee R10 mm punch. The tablets thus obtained were coated with a film coating composition comprising hydroxypropylmethylcellulose, polyethylene glycol 6000, castor oil and methanol to give film-coated tablets.

### Preparation Example 2

| | |
|---|---|
| 3,4,4a,5,8,9,10,10a-octahydro-1,4a-dimethyl-7-(1-methylethyl)-5,8-dioxo-2-phenanthrenecarboxylic acid | 150.0 g |
| Citric acid | 1.0 g |
| Lactose | 33.5 g |
| Dicalcium phosphate | 70.0 g |
| Pluronic F-68 | 30.0 g |
| Sodium lauryl sulfate | 15.0 g |
| Polyvinylpyrrolidone | 15.0 g |
| Polyethylene glycol (Carbowax 1500) | 4.5 g |
| Polyethylene glycol (Carbowax 6000) | 45.0 g |
| Corn starch | 30.0 g |
| Dry sodium lauryl sulfate | 3.0 g |
| Dry magnesium stearate | 3.0 g |
| Ethanol | q.s. |

The above active ingredient compound, citric acid, lactose, dicalcium phosphate, pluronic F-68 and sodium lauryl sulfate were admixed. After size selection using a No. 60 screen, the mixture was granulated by the wet process using an alcoholic solution containing polyvinylpyrrolidone, Carbowax 1500 and Carbowax 6000. When necessary, alcohol was added to make the powder into a paste-like mass. Then, corn starch was added and the blending was continued until uniform granules were formed. The mixture was then passed through a No. 10 screen, placed in a tray and dried in an oven maintained at 100°C for 12 to 14 hours. The dried granules were sieved through a No. 16 screen, then dry sodium lauryl sulfate and dry magnesium stearate were added and, after blending, the mixture was compressed to a desired shape using a tableting machine to give cores.

The above cores were treated with a varnish, dusted with talc for preventing absorption of moisture and then provided with an undercoat layer. Varnish coating was repeated as many times as sufficient for internal use. The tablets were repeatedly applied with a further undercoat layer and a smooth coating. Coloring coating was conducted until a desired coloring was obtained. The coated tablets were obtained by drying.

Pharmacological tests, described hereinafter, were performed on the active ingredient compound of the present invention.

### Pharmacological Test Example 1

### Apoptosis inducing efficacy test

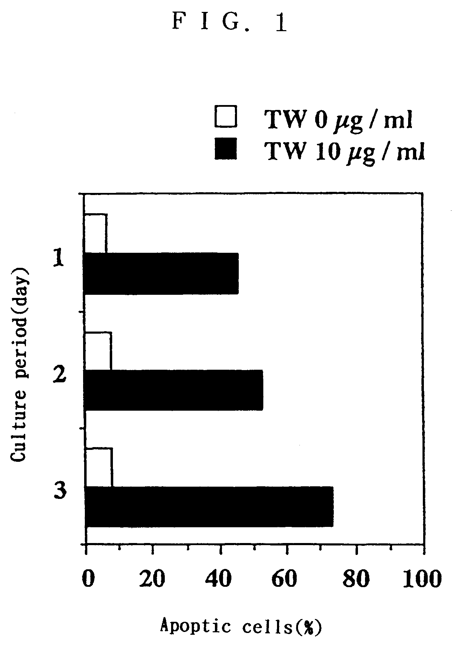

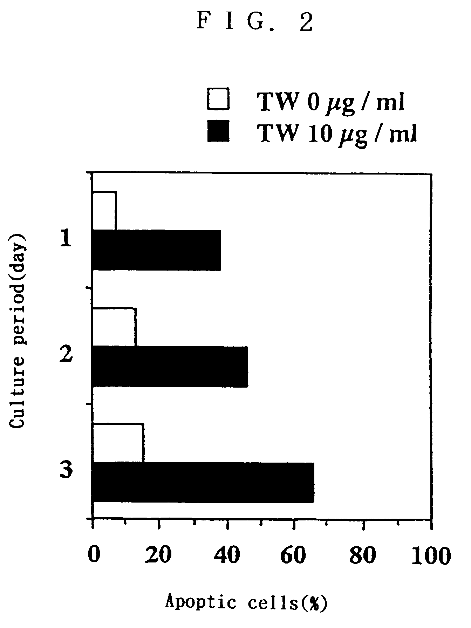

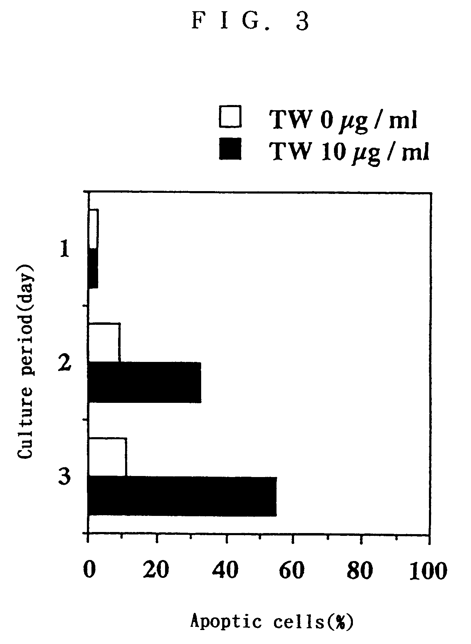

(1) This test was performed in accordance with the method described in the literature [Nicoletti et al., J. Immunological Methods, 139, 271-279 (1991)].
   HL-60 cells were adjusted to a concentration of 5 x 10⁴ cells/ml in RPMI-1640 medium supplemented with 10% fetal calf serum. The compound (A) as the test compound was added to the cell suspension to a concentration of 10 µg/ml. The system mixture was incubated in the wells of a 6-well plate (Costar) at 37°C for 1, 2 or 3 days (test group). As a control, the solvent alone was added to a similar suspension and the mixture was incubated in the same manner (control group). After completion of cultivation, the cells were recovered and 1 x 10⁶ cells were transferred into a polyethylene tube and centrifuged at 200 x g for 5 minutes to provide pellets.
   The pellets obtained above were resuspended in 0.25 ml of buffer [50 µg/ml of propidium iodide (PI, Sigma, 100 µg/ml in PBS) in 0.1% sodium citrate (Wako Pure Chemical) + 0.1% Triton X-100 (Katayama Kagaku)]. The tube was allowed to stand in the dark at 4°C overnight.
   Next day, the PI fluorescence of individual nuclei was determined by a flow cytometer (Profile II, product of Coulter). The flow velocity ratio was set at about 200 nuclei/second and each sample was analyzed for at least 10⁴ nuclei.
   Cell fragments with low intensities of fluorescense were excluded by histogram analysis. Then, the ratio of the nuclei showing a content of not less than 2N to the nuclei showing a content of not more than 2N was calculated. The nuclei showing a content of not more than 2N were regarded as nuclei having apoptic cells and the proportion of nuclei showing a content of not more than 2N was regarded as fragmented DNA and used as an indicator of apoptosis inducing activity.
   The results obtained are shown in Fig. 1.
   In Fig. 1, the culture period (day) was plotted as ordinate and the apoptic cells (%) were plotted as abscissa. The black column is for the test group and the white column for the control group.
   Fig. 1 shows the induction and promotion of apoptosis accomplished by the active ingredient compounds of the invention.
   The human promyelocytic leukemia cell line (HL-60) used in the above test is a human leukemia cell line established by Robert Gallo et al. and typical properties thereof are described in the literature [Gallo, R.C., et al, Blood, 54, 713 (1979)]. The cell line was deposited at the American Type Culture Center (ATCC) under the accession number ATCC No. CCL-240.
(2) A test was carried out in the same manner as in the test (1) using Daudi cells (ATCC No.CCL-213) derived from human Burkitt lymphoma and MOLT-4 cells (ATCC No.CRL-1582) derived from human acute lymphoblastic leukemia in place of HL-60 cells used in the test (1). The results are shown in Figs. 2 and 3 constructed in the same manner as in Fig. 1.
   Figs. 2 and 3, like Fig. 1, show the induction and promotion of apoptosis accomplished by the active ingredient compounds of the invention. (3) It was confirmed by the MTT method [J. Immunol. Methods, 65, 55 (1983)] that the proliferation of said three types of cells was inhibited by the active ingredient of the invention at a concentration of 10 µg/ml.

### Possibility of Use in Industrial Field

The apoptosis inducing composition of this invention has an ability to induce or promote apoptosis and, owing to this ability, is effective in the medicinal field as an anticancer agent, therapeutic agents for autoimmune diseases and for liver diseases such as hepatitis and hepatocirrhosis, a tumor metastasis inhibiting agent, etc.

## Claims

1. An apoptosis inducing composition comprising, as an active ingredient, a pharmacologically effective amount of at least one member selected from the group consisting of phenanthrene derivatives represented by the following formula (1) 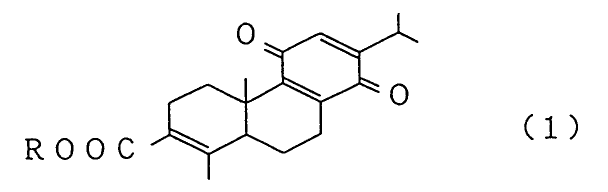 wherein R is a hydrogen atom or a lower alkyl group and salts thereof, and a nontoxic pharmaceutically acceptable carrier therefor.

2. The apoptosis inducing composition according to claim 1 which is used as an anticancer composition in treating a cancer.
